Europäisches Patentamt
European Patent Office
Office européen des brevets

(19)

(11) Veröffentlichungsnummer: **0 053 744**
**A1**

(12) # EUROPÄISCHE PATENTANMELDUNG

(21) Anmeldenummer: 81109767.4

(22) Anmeldetag: 19.11.81

(51) Int. Cl.³: C 07 D 451/06
A 61 K 31/46

(30) Priorität: 04.12.80 DE 3045688

(43) Veröffentlichungstag der Anmeldung: 16.06.82 Patentblatt 82/24

(84) Benannte Vertragsstaaten: AT BE CH DE FR IT LI LU NL SE

(71) Anmelder: C.H. BOEHRINGER SOHN
Postfach 200
D-6507 Ingelheim am Rhein(DE)

(72) Erfinder: Langbein, Adolf, Dr.
Am Goldberg 6
D-6535 Gau-Algesheim(DE)

(72) Erfinder: Merz, Herbert, Dr.
Rotweinstrasse 53
D-6507 Ingelheim/Rhein(DE)

(72) Erfinder: Sobotta, Rainer, Dr.
Fichtenweg 17
D-6507 Ingelheim/Rhein(DE)

(72) Erfinder: Bauer, Rudolf, Dr.
Aarstrasse 4
D-6200 Wiesbaden(DE)

(72) Erfinder: Jennewein, Hans Michael, Prof.Dr.
Gutnussberg
D-6229 Walluf(DE)

(72) Erfinder: Mierau, Joachim, Dr.
An den Weiden 1
D-6500 Mainz 33(DE)

(54) **Neue 8-Arylalkyl-3-phenyl-3-nortropanole, deren Säureadditionssalze, diese enthaltende Arzneimittel und Verfahren zu ihrer Herstellung.**

(57) Die Erfindung betrifft 8-Arylalkyl-3-phenyl-3-nortropanole der allgemeinen Formel I

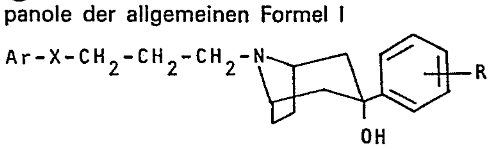
1

worin

Ar einen Rest der Teilformeln - 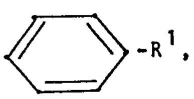 , 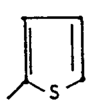 ,

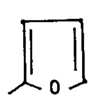 oder - 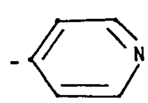

bedeutet,
wobei $R^1$ Wasserstoff, Fluor, Brom, Methyl oder Methoxy ist;
und

X die Reste >CO, >CH-CN, >CHOH, 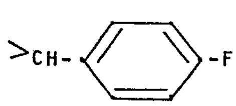 ,

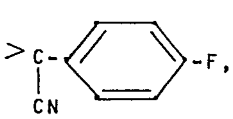 , 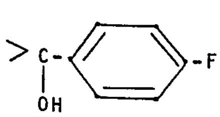 oder -O-, -S- oder

./...

EP 0 053 744 A1

−NH− bedeutet; und

R Wasserstoff oder 4-Fluor, 4-Chlor, 4-Trifluormethyl, 3-Trifluormethyl, 3-Trifluormethyl-4-chlor, 4-Methyl oder 4-Methoxy bedeutet und deren Säureadditionssalze.

Die Verbindungen können durch Umsetzung eines 3-Phenyl-3-nortropanols der allgemeinen Formel

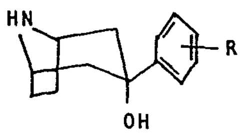

II

mit einem Alkylierungsmittel der allgemeinen Formel

$$Ar-X-CH_2-CH_2-CH_2-Y \quad (III)$$

hergestellt werden.

Die Verbindungen wirken ZNS-dämpfend und sind als Neuroleptika verwendbar.

Gegenstand der Erfindung sind neue 8-Arylalkyl-3-phenyl-3-nortropanole der allgemeinen Formel

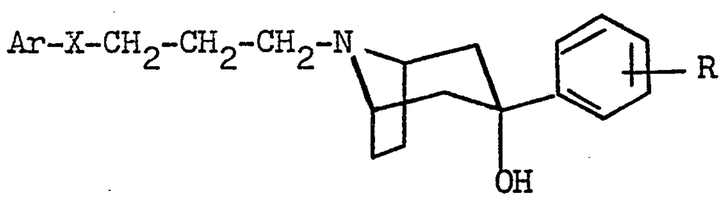 I

sowie deren physiologisch verträgliche Säureadditionssalze mit wertvollen therapeutischen Eigenschaften. In der allgemeinen Formel I bedeutet

Ar einen Rest der Teilformel 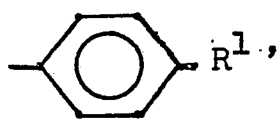 , 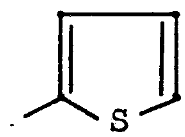 ,

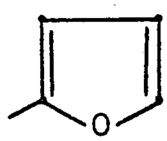 oder 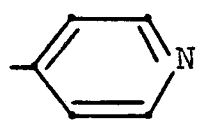

wobei

$R^1$ Wasserstoff, Fluor, Brom, Methyl oder Methoxy ist; und

X die Reste >CO , >CH-CN, >CHOH, 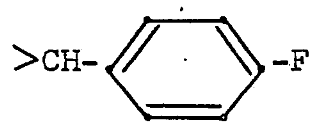 ,

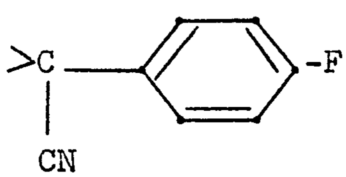 , 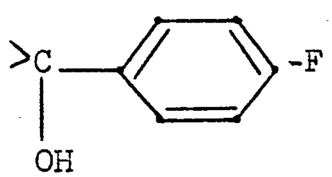 oder -O-, -S- oder

-NH- ; und

R Wasserstoff oder 4-Fluor, 4-Chlor, 4-Trifluormethyl, 3-Trifluormethyl, 3-Trifluormethyl-4-chlor, 4-Methyl oder 4-Methoxy.

Bevorzugt sind Verbindungen der allgemeinen Formel I, worin Ar einen 4-Fluorphenylrest, X die Reste >CO, >CH-CN, >CHOH oder

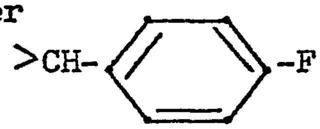

und R ein Halogenatom bedeutet. Ganz besonders bevorzugt sind Verbindungen der allgemeinen Formel I, worin

Ar einen 4-Fluorphenylrest, X >CO, >CH-CN und R ein Halogenatom bedeutet.

Verbindungen der allgemeinen Formel I, worin X die Reste

>CH-CN, >CHOH, 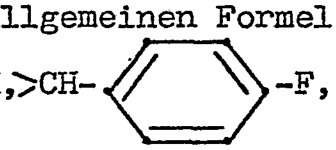, 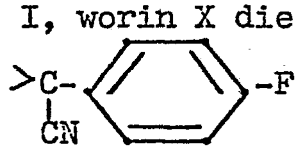 , 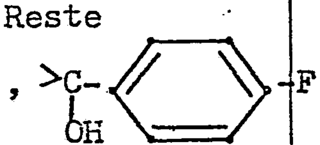

bedeuten, besitzen ein asymmetrisches Kohlenstoffatom und kommen daher sowohl als Racemat als auch in Form ihrer optischen Antipoden vor.

Die Erfindung betrifft weiterhin Verfahren zur Herstellung der Verbindungen der allgemeinen Formel I, wobei folgende Verfahren besonders hervorzuheben sind:

a) die Herstellung der neuen Verbindungen kann durch Umsetzung eines 3-Phenyl-3-nortropanols der allgemeinen Formel II

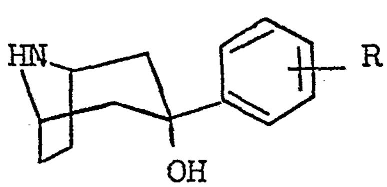

II

worin R die oben genannte Bedeutung hat, mit einem Alkylierungsmittel der allgemeinen Formel III

$$Ar-X-CH_2-CH_2-CH_2-Y \qquad III$$

erfolgen, worin Y ein Halogenatom oder einen Alkylbenzolsulfonyloxyrest bedeutet und Ar und X wie oben angegeben definiert sind.

Besonders bevorzugt sind Verbindungen der Formel III, worin Y ein Chlor- oder Bromatom oder einen Tosyloxyrest darstellt. Man verwendet die berechnete Menge des Alkylierungsmittels oder einen stöchiometrischen Überschuß davon und arbeitet zweckmäßig in Gegenwart eines säurebindenden Stoffes, wie z.B. Triäthylamin, Dicyclohexyläthylamin, Natriumcarbonat, Kaliumcarbonat, Calciumoxyd oder vorzugsweise Natriumhydrogencarbonat.

Obwohl auf Lösungsmittel verzichtet werden kann, ist die Durchführung in inerten Lösungsmitteln,wie Chloroform, Toluol, Äthanol, Nitromethan, Tetrahydrofuran oder vorzugsweise Dimethylformamid zweckmäßiger. Die Reaktionstemperatur ist in weiten Grenzen variabel. Zweckmäßig sind Temperaturen zwischen 50°C und 150°C, vorzugsweise 100°C. Die Zugabe von katalytischen bis molaren Mengen Kaliumjodid oder Natriumjodid erweist sich als günstig.

b) Zur Herstellung von Verbindungen der allgemeinen Formel I, worin X die Gruppen 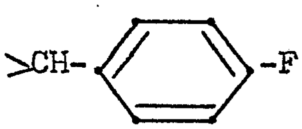 , -O- oder -S- bedeutet,

kann man ein 8-Arylalkyl-nortropin-3-on der allgemeinen Formel

$Ar-X'-CH_2-CH_2-CH_2-N$ 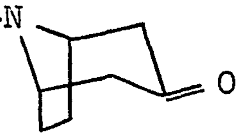 IV

worin X' die Gruppen 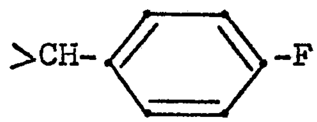 , -O- oder -S- bedeutet

und Ar wie oben angegeben definiert ist, mit einer Lithiumphenyl-Verbindung der allgemeinen Formel

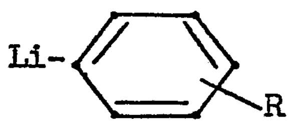

V

umsetzen, worin R die oben genannte Bedeutung hat. Zweckmäßigerweise arbeitet man mit molaren Mengen an Lithiumphenyl-Verbindung und führt die Umsetzung in inerten Lösungsmitteln durch, die keine aktiven Wasserstoffatome oder mit Lithiumarylen umsetzbare Gruppen enthalten, wie Benzol, Toluol, Petroläther oder aliphatische oder cycloaliphatische Äther. Als Äther kommen insbesondere Diäthyläther, Tetrahydrofuran und Dioxan in Frage.

Die Reaktionstemperatur ist kritisch; die besten Ausbeuten werden bei niederen Temperaturen von -40 bis -15°C erhalten.

c) Zur Herstellung von Verbindungen der allgemeinen Formel I, worin X die Gruppe 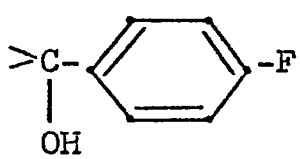 und R 4-Fluor bedeuten, kann

man ein 8-Aroylpropyl-nortropin-3-on der allgemeinen Formel

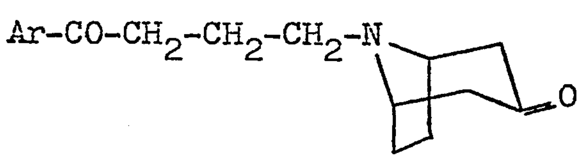

VI

worin Ar die oben angegebene Bedeutung besitzt, mit mindestens 2 Molen 4-Fluorphenyl-lithium umsetzen. Die Reaktionsbedingungen für diese spezielle Form der Arylierung sind die gleichen wie die im Verfahren b genannten.

d) Verbindungen der allgemeinen Formel I, worin X die CO-Gruppe bedeutet, können durch Ketalspaltung erhalten werden, indem man ein 8-Arylalkyl-3-phenyl-3-nortropanol der allgemeinen Formel

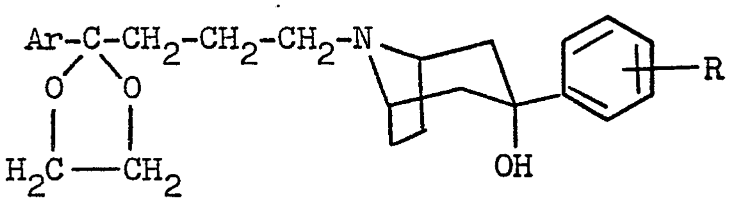

VII

worin Ar und R die oben genannten Bedeutungen besitzen, mit verdünnten Säuren umsetzt.

Die Ketalspaltung wird vorzugsweise in einem wässrigen organischen Lösungsmittel vorgenommen, z.B. einem niederen aliphatischen Alkohol. Als Säuren verwendet man bevorzugt verdünnte Mineralsäuren, wie Halogenwasserstoffsäuren oder Schwefelsäure.

Die nach den Verfahren a, b, c und d erhaltenen Reaktionsprodukte werden aus den Reaktionsansätzen mit Hilfe bekannter Methoden isoliert. Gegebenenfalls können die so erhaltenen Rohprodukte noch unter Anwendung besonderer Verfahren, z.B. durch Säulenchromatographie, gereinigt werden, ehe man sie in Form der Basen oder geeigneter Säureadditionsverbindungen kristallisiert.

Die für die Verfahren a, b und c eingesetzten Ausgangsverbindungen der allgemeinen Formel II, IV und VI können auf folgendem Wege hergestellt werden: Nortropinon der Formel VIII ist nach dem Verfahren der GB-PS 1 167 688 erhältlich. Durch Benzylierung (A) oder Äthoxycarbonylierung (B) erhält man die substituierten Nortropinone der Formel IX und X. Aus diesen werden die entsprechenden Nortropanole der allgemeinen Formeln XI und XII durch Reaktion

mit Lithiumphenylen hergestellt. Als Lösungsmittel dienen Benzol, Toluol, Petroläther oder Äther. Die Reaktionstemperaturen dürfen einen kritischen Bereich nicht überschreiten und liegen bei $-35^{o}$ bis $-15^{o}C$. Die Spaltung zu den Nortropanolen der allgemeinen Formel II erfolgt nach dem Weg A durch katalytische Entbenzyllierung über Metallkatalysatoren wie Palladium/Kohle. Die Lösungsmittel sind in weiten Grenzen variierbar; günstig sind Alkohole wie Methanol, Äthanol oder i-Propanol. Der Druckbereich ist begrenzt auf maximal 5 bar. Der Temperaturbereich ist variabel, sollte aber $50^{o}C$ nicht überschreiten.

Auf dem Weg B werden die Nortropanole der allgemeinen Formel II durch Verseifung der Verbindungen der Formel XII erhalten. Die Verseifungsbedingungen sind in weiten Grenzen variabel. Vorzugsweise verwendet man Kalilauge oder Natronlauge in Alkohol/Wasser-Gemischen. Auch der Temperaturbereich ist unkristisch, vorteilhaft ist Rückflußkochen.

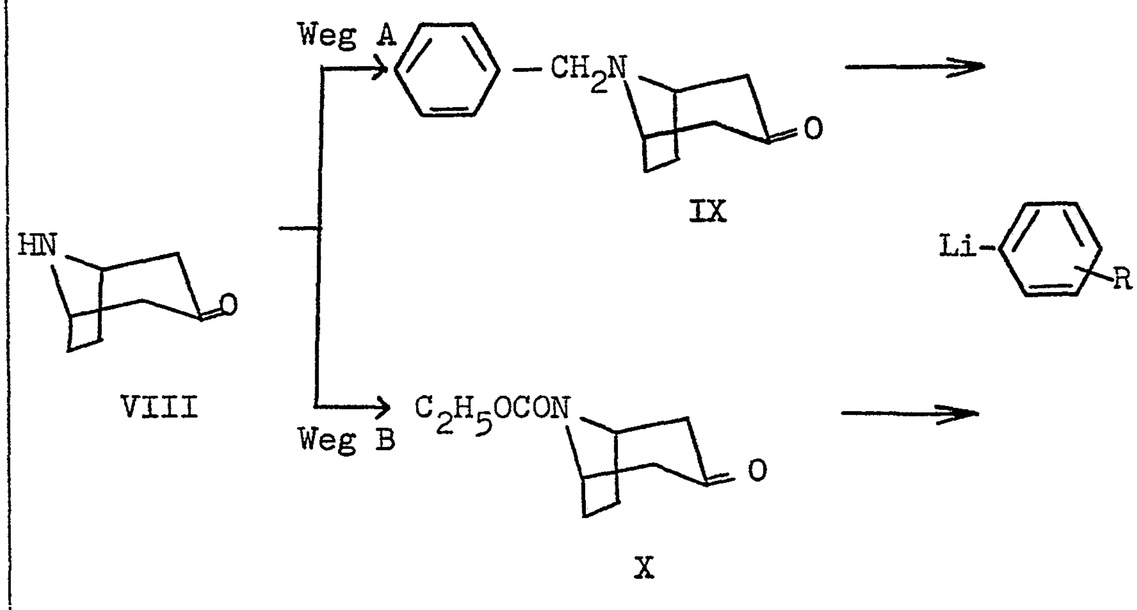


IX

VIII

X

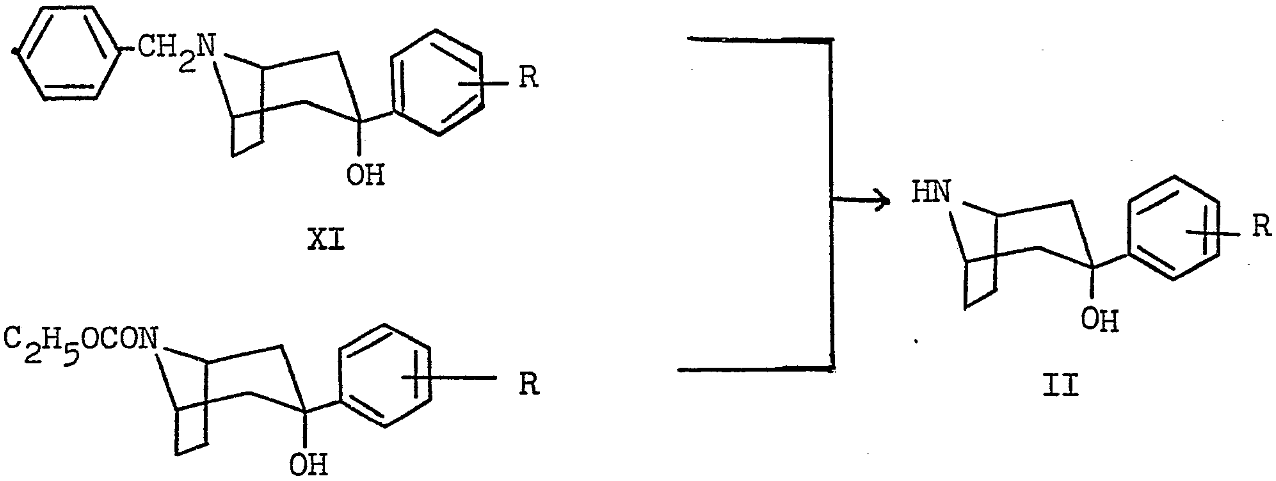

XI

XII

II

Ausgangsverbindungen der Formel VII erhält man nach Verfahren a) durch Alkylierung eines 3-Phenyl-3-nortropanols der allgemeinen Formel II mit einem Alkyierungsmittel der allgemeinen Formel

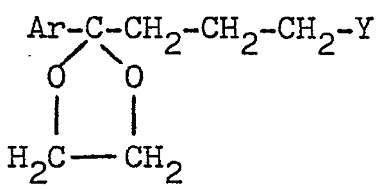

XIII

worin Ar und Y wie oben angegeben definiert sind.

Die erfindungsgemäßen 8-Arylalkyl-3-phenyl-3-nortropanole der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I zeigen das typische Wirkungsbild der Neuroleptika und sollen daher als ZNSdämpfende Mittel, als Sedativa und Tranquilizer eingesetzt werden. Gegenüber dem strukturverwandten, gleichfalls neuroleptisch wirkenden Haloperidol (1-(3-p-Fluorbenzoylpropyl)-4-p-chlorphenyl-4-hydroxypiperidin) zeigen die neuen Verbindungen ein günstigeres Wirkungsprofil.

Die Verbindungen wurden im Apomorphin-Klettertest an der Maus nach B. Costall et al., Europ. J. Pharmacol. 50, 39 ff (1978) untersucht. Sie zeigen starke apomorphinantagonistische Wirkungen, die die Wirkungen von Haloperidol vielfach beträchtlich übertreffen. Diese Eigenschaften lassen nach dem Stand der Technik neuroleptische Wirkungen am Menschen erwarten. Aussagen über Nebenwirkungen wie Sedation oder Störungen der Motokoordination konnten mit Hilfe folgender Testanordnungen gemacht werden: Die Motilitätsprüfung an Mäusen nach T.H. Svensson und G. Thieme, Psychopharmacol. (Berlin) 14, 157 ff (1969) gibt Hinweise auf Sedation. Der Ataxietest am rotierenden Stab (Rotarod) bei Mäusen nach N.W. Dunham und T.S. Miya, J. Amer. Pharm. Assoc. Sci. Ed., 46, 208 ff (1957) erlaubt Aussagen über Störungen der Motokoordination. Bezüglich der Nebenwirkungen verhalten

sich die erfindungsgemäßen Verbindungen günstiger als die Vergleichssubstanz Haloperidol.

Zur biochemischen Charakterisierung der erfindungsgemäßen Verbindungen dienten Bindungsstudien an Ratten. So wurden die Substanzen im $^3$H-Spiroperidol-Bindungstest (Bindungstest mit tritium-markiertem 8-[3-(p-Fluorbenzoyl)-propyl]-1-phenyl-1,3,8-triazaspiro[4,5]decan-4-on) an Synaptosomen-Präparationen aus Rattenhirn (Striatum) nach J. Creese, D.R. Burt und S.H. Snyder, Science 188, 1217 (1975) untersucht. Sie zeigen ein gegenüber dem Haloperidol vielfach besseres Bindungsverhalten und sind somit als stark wirksame Neuroleptika charakterisiert. Außerdem erfolgten Untersuchungen über α-adrenerge und α-adrenolytische Eigenschaften mit Hilfe von tritium-markiertem 2-[2-(2',6'-Dimethoxy-phenoxy)-ethyl-amino-methyl]-benzodioxanhydrochlorid, einen α-adrenergen Antagonisten. In diesem Bindungstest zeigen die erfindungsgemäßen Verbindungen vielfach dem Haloperidol überlegene Eigenschaften (D.C. U'Prichard, D.A. Greenberg und S.H. Snyder, Mol. Pharmacol. 13, 454 ff (1977)). Untersuchungen auf muscarinisch anticholinerge Bindungseigenschaften wurden in Bindungstesten mit tritium-markiertem 3-Quinuclidinyl-benzilat nach Yamamura und S.H. Snyder, Proceed. Nat. Acad. Sci. USA, 71, 1725 - 1729 (1974) durchgeführt. Hier zeigen die erfindungsgemäßen Verbindungen teilweise stärkere anticholinerge Bindungseigenschaften als Haloperidol.

Die erfindungsgemäßen Verbindungen der allgemeinen Formel I können allein oder in Kombination mit anderen Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen,beispielsweise inerten Verbindungsmitteln wie Calciumcarbonat, Calciumphosphat oder Milchzucker, Sprengmitteln wie Maisstärke,Alginsäure, Bindemitteln wie Stärke oder Gelatine, Schmiermitteln wie Magnesiumstearat oder Talkum und/oder Mitteln

zur Erzielung eines Depoteffektes wie Carboxypolymethylen, Carboxymethylcellulose, Celluloseacetatphthalat oder Polyvinylacetat erhalten werden.

Die neuen Verbindungen der allgemeinen Formel I könnnen enteral oder parenteral angewandt werden. Bei oraler Verabreichung haben sich Einzeldosierungen von 0,5 - 10 mg, vorzugsweise 1 - 5 mg als zweckmäßig erwiesen.

Die folgenden Beispiele erläutern die Erfindung in nicht beschränkender Weise.

A. Vorprodukte und Ausgangsverbindungen

8-Benzylnortropinon

161,6 g (1 Mol) Nortropinonhydrochlorid (nach GB-PS 1 167 688 aus dem Tropinon durch Phosgen-Entmethylierung hergestellt), 139,2 g (1,1 Mol) Benzylchlorid, 336 g (4 Mol) Natriumbicarbonat und 16,6 g (0,1 Mol) Kaliumjodid werden in 1600 ml absolutem Dimethylformamid 1,5 Stunden bei 100°C gerührt. Anschließend wird das Lösungsmittel abdestilliert, der Rückstand in 1,5 Liter Essigester suspendiert und dreimal mit je 500 ml Wasser kräftig geschüttelt. Die Essigesterphase wird über Magnesiumsulfat getrocknet, abfiltriert und eingeengt. Das verbleibende Öl löst man in 700 ml Äthanol und versetzt die Lösung mit 390 ml 2,5 N äthanolischer Salzsäure. Das auskristallisierte Salz wird nach dem Abkühlen abgesaugt, einmal mit 50 ml eiskaltem Äthanol und dreimal mit je 50 ml Äthanol/Äther 1:1 gewaschen. Es werden 201,4 g weiße Kristalle in einer Ausbeute von 80 % der Theorie mit einem Schmelzpunkt von 195 - 196°C gewonnen.

| | | | | | |
|---|---|---|---|---|---|
| $C_{14}H_{17}NO$ x HCl | ber. | C 66,79 | H 7,21 | N 5,56 | Cl 14,08 |
| (MW: 251,75) | gef. | 66,40 | 7,58 | 5,06 | 14,11 |

8-Carbäthoxinortropinon

161,6 g (1 Mol) Nortropinon-hydrochlorid werden mit 252,7 g (2,5 Mol) Triäthylamin in 1450 ml absolutem Methylenchlorid

vorgelegt und mit 119,4 g (1,1 Mol) Chlorameisensäureäthylester, gelöst in 600 ml absolutem Methylenchlorid, tropfenweise innerhalb von 30 min versetzt. Dabei steigt die Temperatur auf 40°C. Die Mischung wird 4 Stunden bei Zimmertemperatur nachgerührt. Anschließend wird die Suspension einmal mit 500 ml Wasser unter Zufügen von etwas Eis, zweimal mit je 500 ml verdünnter Salzsäure und nochmals zweimal mit je 500 ml Wasser kräftig geschüttelt. Die organische Phase saugt man nach dem Trocknen mit Magnesiumsulfat ab und filtriert die braune Lösung über 50 g Kieselgel. Am Rotationsverdampfer wird dann bei 40°C eingeengt. Es bleiben 178,5 g eines farblosen Öles in einer Ausbeute von 90,5 %, das sofort weiter umgesetzt wird.

8-Benzyl-3-(4-trifluormethylphenyl)-3-nortropanol-methansulfonat

Zu 100 ml einer 1,6 N Lithiumbutyllösung in Hexan (0,16 Mol) werden unter Stickstoff bei -35° C in 200 ml Äther innerhalb von 10 min 38,3 g p-Brombenzotrifluorid (0,17 Mol) in 100 ml Äther getropft. Nach 5 min gibt man sehr schnell 25,8 g (0,12 Mol) 8-Benzylnortropinon in 150 ml Äther bei -40°C dazu. Das Reaktionsgemisch bleibt 1 Stunde bei -40 bis -10°C und wird anschließend vorsichtig auf 200 ml Wasser gegeben, das 10 g Ammoniumchlorid enthält. Die Ätherphase wird abgetrennt, einmal mit 100 ml einer 1 N Ammoniumchloridlösung und zweimal mit je 100 ml Wasser gewaschen. Nach dem Trocknen über Magnesiumsulfat wird die Lösung abgesaugt, über 200 g Kieselgel filtriert und eingeengt. Der verbleibende Rückstand wird in 25 ml Äthanol gelöst, mit molarer Menge alkoholischer Methansulfonsäure und wenig Äther versetzt. Nach einiger Zeit kristallisiert das Methansulfonat aus, das filtriert und mit Alkohol/Äther gut gewaschen wird. Man erhält auf diese Weise 37,3 g des Methansulfonates entsprechend einer Ausbeute von 68 % der Theorie mit einem Schmelzpunkt von 250°C.

| | | | | | |
|---|---|---|---|---|---|
| $C_{21}H_{22}F_3NO \times CH_3SO_3H$ | ber. | C 57,76 | H 5,73 | F 12,46 | N 3,06 |
| (MW: 457,44) | gef. | 57,63 | 5,51 | 12,50 | 2,86 |

Auf diese Weise wurden die folgenden Verbindungen hergestellt:

| | Fp. °C |
|---|---|
| 8-Benzyl-3-phenyl-3-nortropanol-methansulfonat | 220° |
| 8-Benzyl-3-(4-fluorphenyl)-3-nortropanol-methan-sulfonat | 221° |
| 8-Benzyl-3-(4-methylphenyl)-3-nortropanol-hydro-chlorid | 237° |
| 8-Benzyl-3-(4-methoxyphenyl)-3-nortropanol-hydro-chlorid | 196° |
| 8-Benzyl-3-(3-trifluormethylphenyl)-3-nortropanol-methansulfonat | 229 - 230° |

8-Carbäthoxi-3-(4-chlorphenyl)-3-nortropanol

Man legt 100 ml einer 1,6 molaren Butyllithium-Hexanlösung in 200 ml absolutem Äther vor. Bei -35°C und unter Stickstoff werden innerhalb von 10 min 32,5 g (0,17 Mol) p-Chlorbrombenzol in 100 ml Äther dazugetropft. Nach 5 min Rühren gibt man 23,7 g (0,12 Mol) frisch bereitetes 8-Carbäthoxinortropinon in 150 ml Äther sehr schnell dazu. Dabei steigt die Temperatur auf -15°C. Die Suspension wird wieder auf -35°C abgekühlt und erst innerhalb von 1 1/2 Stunden auf +10°C erwärmt. Die Lösung zersetzt man vorsichtig mit 200 ml Wasser. Dabei wird mit 10 g Ammoniumchlorid abgepuffert. Die Ätherphase wäscht man noch dreimal mit jeweils 100 ml Wasser. Anschließend wird die organische Phase mit Magnesiumsulfat getrocknet, abgesaugt und am Rotationsverdampfer bei 40°C eingeengt. In 250 ml Methylenchlorid/Methanol (98/2) löst sich der Rückstand, der über 500 g Kieselgel filtriert und eingeengt wird. Der Rückstand kristallisiert aus Äther/Petroläther (40/60) und ergibt 13,6 g weiße Kristalle entsprechend einer Ausbeute von 36,6 % mit einem Schmelzpunkt von 115°C.

| | | | | | |
|---|---|---|---|---|---|
| $C_{16}H_{20}ClNO_3$ | ber. | C 62,03 | H 6,51 | Cl 11,45 | N 4,52 |
| (MW: 309,79) | gef. | 62,32 | 6,65 | 11,40 | 4,47 |

Entsprechend wurden folgende Verbindungen erhalten:

| | Fp. °C |
|---|---|
| 8-Carbäthoxi-3-(4-chlor-3-trifluormethylphenyl)-3-nortropanol | 167° |
| 8-Carbäthoxi-3-phenyl-3-nortropanol | 104° |

3-(4-Trifluormethylphenyl)-3-nortropanol-methansulfonat

14 g (0,031 Mol) 8-Benzyl-3-(4-trifluormethylphenyl)-3-nortropanol-methansulfonat werden in 140 ml Methanol gelöst, mit 1,4 g 5 % Palladium/Kohle versetzt und bei 5 bar und 50°C hydriert. Nach 2 Stunden ist die Wasserstoffaufnahme beendet. Die warme Lösung wird abgesaugt und am Rotationsverdampfer bei 50°C eingeengt. Der Rückstand kristallisiert aus Isopropanol. Dabei erhält man 9,6 g weiße Kristalle entsprechend einer Ausbeute von 85,4 % mit einem Schmelzpunkt von 273°C.

| | | | | | |
|---|---|---|---|---|---|
| $C_{14}H_{16}F_3NO$ x $CH_3SO_3H$ | ber. | C 49,04 | H 5,49 | F 15,52 | N 3,81 |
| (MW: 367,38) | gef. | 48,85 | 5,22 | 15,76 | 3,61 |

Analog wurden die folgenden Verbindungen erhalten:

| | Fp. °C |
|---|---|
| 3-Phenyl-3-nortropanol-methansulfonat | 216 - 217° |
| 3-(4-Fluorphenyl)-3-nortropanol-methansulfonat | 229° |
| 3-(4-Methylphenyl)-3-nortropanol-hydrochlorid | 252° |
| 3-(4-Methoxiphenyl)-3-nortropanol-hydrochlorid | 216° |
| 3-(3-Trifluorphenyl)-3-nortropanol-methansulfonat | 207° |

3-(4-Chlorphenyl)-3-nortropanol-hydrochlorid

18,6 g (0,06 Mol) 8-Carbäthoxi-3-(4-chlorphenyl)-3-nortropanol werden zusammen mit 19,4 g (0,35 Mol) Kaliumhydroxid in einem Gemisch aus 190 ml i-Propanol und 12,5 ml Wasser 70 Stunden unter Rückfluß gerührt. Dann versetzt man mit 200 ml Wasser und neutralisiert mit Eisessig. Nach 1/2 Stunde Rühren engt

man im Vakuum am Rotationsverdampfer ein. Der Rückstand löst sich in 150 ml 10 %iger Methansulfonsäure, die Lösung wird zweimal mit je 100 ml Äther geschüttelt. Aus dieser Ätherphase können 9 g Ausgangsverbindung zurückerhalten werden.

Die saure wäßrige Phase ergibt nach Alkalischstellen mit konzentrierter Kalilauge und Ausschütteln mit dreimal je 150 ml Methylenchlorid, das 5 % Butanol enthält, einen Rückstand. Dieser wird in Äthanol gelöst und mit molarer Menge äthanolischer Salzsäure versetzt. Nach Zugabe von wenig Äther kristallisieren 6,3 g Hydrochlorid in einer Ausbeute von 38,3 % mit einem Schmelzpunkt von 271°C.

| | | | | | |
|---|---|---|---|---|---|
| $C_{13}H_{16}ClNO$ x HCl | ber. | C 56,94 | H 6,25 | Cl 25,86 | N 5,11 |
| (MW: 274,19) | gef. | 57,10 | 6,29 | 25,87 | 5,12 |

Analog wurden folgende Verbindungen erhalten:

| | Fp. °C |
|---|---|
| 3-Phenyl-3-nortropanol-hydrochlorid | 216 - 217° |
| 3-(4-Chlor-3-trifluormethylphenyl)-3-nortropanol-methansulfonat | 195° |

N-(4,4-Bis-4'-Fluorphenylbutyl)nortropinon-hydrochlorid

16,1 g (0,1 Mol) Nortropinonhydrochlorid werden zusammen mit 30,9 g (0,11 Mol) 4,4-Bis-4'-Fluorphenylbutylchlorid, 25,4 g (0,3 Mol) Natriumbicarbonat und 5 g Kaliumjodid in 250 ml Dimethylformamid 4 Std. auf 100°C unter Rühren erhitzt. Danach wird das Lösungsmittel am Rotationsverdampfer bei 70°C entfernt. Der Rückstand wird in 200 ml Essigester aufgenommen und mit je 100 ml Wasser zweimal gewaschen. Die organische Phase wird über Natriumsulfat getrocknet und über 10 g Kieselgel filtriert. Anschließend engt man am Rotationsverdampfer ein. Der Rückstand wird in Äthanol gelöst und mit 0,1 Mol äthanolischer Salzsäure in leichtem Überschuß versetzt. Es kristallisiert

das Hydrochlorid, das einmal aus Äthanol umkristallisiert wird. Man erhält 18,6 g obiger Verbindung in einer Ausbeute von 45,7 % mit einem Schmelzpunkt von 210°C.

| | | | | | | |
|---|---|---|---|---|---|---|
| $C_{23}H_{25}F_2NO$ x HCl | ber. | C 68,05 | H 6,46 | Cl 8,74 | N 3,45 |
| (MW: 405,90) | gef. | 68,02 | 6,60 | 8,84 | 3,42 |

N-(4-Fluorphenyl-4-oxobutyl)-nortropinon-hydrochlorid

16,2 g (0,1 Mol) Nortropinon-hydrochlorid werden zusammen mit 26,9 g (0,11 Mol) Äthylenketal des 4-Chlor-4'-fluorphenyl-butyrophenon, 25,4 g (0,3 Mol) Natriumbicarbonat und 5 g Kaliumjodid in 250 ml Dimethylformamid 3 Std. auf 100°C unter Rühren erhitzt. Danach wird das Lösungsmittel am Rotationsverdampfer bei 70°C entfernt. Der Rückstand wird in 200 ml Essigester aufgenommen und mit je 100 ml Wasser zweimal gewaschen, getrocknet und eingeengt. Den Rückstand löst man in 500 ml 2 N Salzsäure unter Zusatz von 500 ml Äthanol und erwärmt 1 Stunde auf 50°C unter Rühren. Anschließend wird die Lösung bei 50°C am Rotationsverdampfer eingeengt.

Man stellt unter Eiskühlung mit 50 ml konzentriertem Ammoniak alkalisch und schüttelt 3 mal mit je 100 ml Methylenchlorid aus. Die organische Phase wird über Natriumsulfat getrocknet und über 10 g Kieselgel filtriert. Anschließend engt man am Rotationsverdampfer ein. Der Rückstand wird in Äthanol gelöst, mit 0,1 Mol äthanolischer Salzsäure in leichtem Überschuß versetzt und das Hydrochlorid kristallisiert. Man saugt ab und kristallisiert nochmals aus Äthanol um, wobei man 17,5 g obiger Verbindung in einer Ausbeute von 53,8 % mit einem Schmelzpunkt von 173°C erhält.

| | | | | | |
|---|---|---|---|---|---|
| $C_{17}H_{20}FNO_2$ x HCl | ber. | C 62,67 | H 6,50 | F 5,83 | N 4,30 |
| (MW: 325,81) | gef. | 62,46 | 6,47 | 5,82 | 4,23 |

## B. Herstellungsbeispiele

### Beispiel 1 (Verfahren a und d)

**8-(4-p-Fluorphenyl-4-oxobutyl)-3-(4-chlorphenyl)-3-nortropanol-hydrochlorid**

27,4 g (0,1 Mol) 3-(4-Chlorphenyl)-3-nortropanolhydrochlorid werden zusammen mit 26,9 g (0,1 Mol) Äthylenketal des 4-Chlor-4'-fluorphenylbutyrophenon, 25,4 g (0,3 Mol) Natriumbicarbonat und 5 g Kaliumjodid in 250 ml Dimethylformamid 5 Stunden auf 100°C unter Rühren erhitzt. Danch wird das Lösungsmittel am Rotationsverdampfer bei 70°C entfernt. Der Rückstand wird in 200 ml Essigester aufgenommen, mit je 100 ml Wasser zweimal gewaschen und eingeengt. Der Rückstand wird in 500 ml 2 N Salzsäure und 500 ml Äthanol gelöst. Die Salzsäure-Lösung erwärmt man 30 min auf 50°C. Nach dem Abkühlen engt man ein. Den Rückstand stellt man unter Eiskühlung mit 50 ml konzentiertem Ammoniak alkalisch und schüttelt dreimal mit je 100 ml Methylenchlorid aus. Die organische Phase wird über Natriumsulfat getrocknet und über 10 g Kieselgel filtriert. Anschließend engt man am Rotationsverdampfer ein. Der Rückstand wird in 50 ml Äthanol gelöst, mit 0,1 Mol äthanolischer Salzsäure und dann mit Äther versetzt, so daß die Trübung gerade wieder verschwindet. Man erhält 24,5 g obiger Verbindung in einer Ausbeute von 56 % mit einem Schmelzpunkt von 244°C.

| | | | | | | | |
|---|---|---|---|---|---|---|---|
| $C_{23}H_{25}ClFNO_2$ x HCl | ber. | C 63,01 | H 5,98 | Cl 16,18 | F 4,33 | N 3,20 |
| (MW: 438,36) | gef. | 62,88 | 5,64 | 15,76 | 4,28 | 2,91 |

Analog dem Beispiel 1 wurden die in der Tabelle 1 aufgeführten Verbindungen hergestellt. Sie leiten sich von der allgemeinen Formel ab.

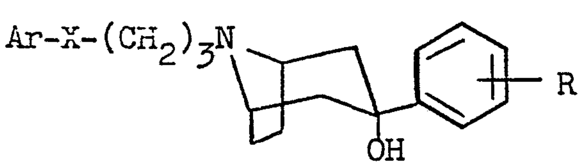

I

Tabelle 1: Verbindungen der allgemeinen Formel I

| Beispiel | Ar | X | R | Schmelzpunkt: °C | Salz |
|---|---|---|---|---|---|
| 2 | 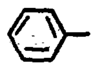 | >CO | H | 228 | HCl |
| 3 | 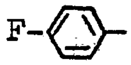 | >CO | H | 229 | HCl |
| 4 | 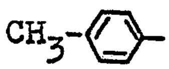 | >CO | H | 217 | HCl |
| 5 | 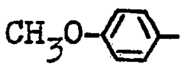 | >CO | H | 211 - 212 | HCl |
| 6 | 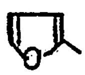 | >CO | H | 268 | HCl |
| 7 | 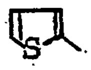 | >CO | H | 249 | HCl |
| 8 | 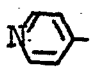 | >CO | H | 201 - 203 | 2HCl |
| 9 | 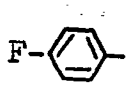 | 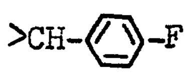 | H | 237 | HCl |
| 10 | 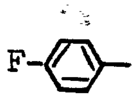 | >CH-C≡N | H | 199 - 200 | HCl |
| 11 | 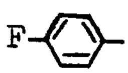 | -O- | H | 143 | HCl |
| 12 | 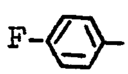 | -S- | H | 193 - 194 | HCl |
| 13 | 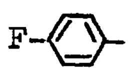 | -NH- | H | 160 | 2HCl |
| 14 | 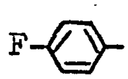 | >CH-OH | H | 146 - 148 | HCl |
| 15 | 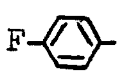 | 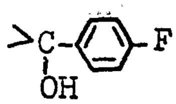 | H | 202 | HCl |
| 16 | 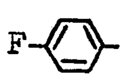 | 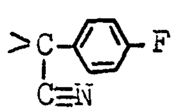 | H | 265 - 266 | HCl |

| Beispiel | Ar | X | R | Schmelzpkt.: °C | Salz |
|---|---|---|---|---|---|
| 17 | 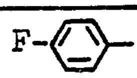 | >CO | 4-$CF_3$ | 210 | $CH_3SO_3H$ |
| 18 | 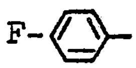 | >CH-C≡N | 4-$CF_3$ | 144 | $CH_3SO_3H$ |
| 19 | 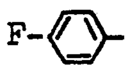 | 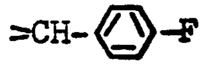 | 4-$CF_3$ | 252 | $CH_3SO_3H$ |
| 20 | 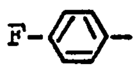 | >CH-OH | 4-$CF_3$ | 232 | HCl |
| 21 | 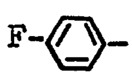 | 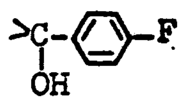 | 4-$CF_3$ | 262 | HCl |
| 22 | 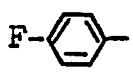 | 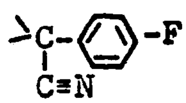 | 4-$CF_3$ | 280 | HCl |
| 23 | 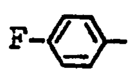 | >CO | 4-F | 226 | $CH_3SO_3H$ |
| 24 | 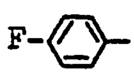 | >CH-CN | 4-F | 199 | $CH_3SO_3H$ |
| 25 | 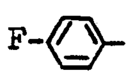 | 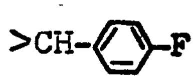 | 4-F | 225 | $CH_3SO_3H$ |
| 26 | 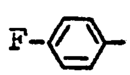 | >CH-OH | 4-F | 227 | HCl |
| 27 | 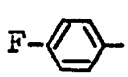 | 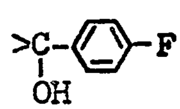 | 4-F | 215 | HCl |
| 28 | 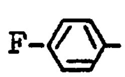 | 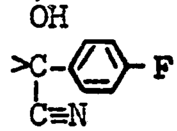 | 4-F | 259 | HCl |
| 29 | 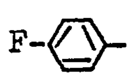 | >CH-CN | 4-Cl | 173 | $CH_3SO_3H$ |
| 30 | 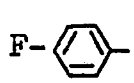 | 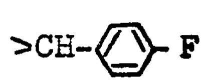 | 4-Cl | 127 | Base |

| Beispiel | Ar | X | R | Schmelzpkt.: °C | Salz |
|---|---|---|---|---|---|
| 31 | 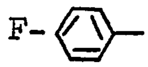 | -O- | 4-Cl | 238 | $CH_3SO_3H$ |
| 32 | 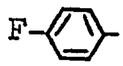 | -S- | 4-Cl | 204 | $CH_3SO_3H$ |
| 33 | 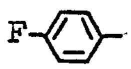 | -NH- | 4-Cl | 196 | 2HCl |
| 34 | 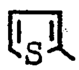 | >CO | 4-Cl | 197 | $CH_3SO_3H$ |
| 35 | 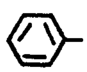 | >CO | 4-Cl | 228 - 229 | HCl |
| 36 | 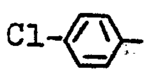 | >CO | 4-Cl | 273 | HCl |
| 37 | 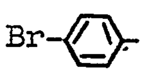 | >CO | 4-Cl | 220 | $CH_3SO_3H$ |
| 38 | 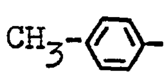 | >CO | 4-Cl | 227 | $CH_3SO_3H$ |
| 39 | 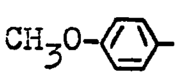 | >CO | 4-Cl | 205 | $CH_3SO_3H$ |
| 40 | 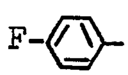 | >CH-OH | 4-Cl | 219 | HCl |
| 41 | 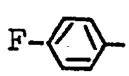 | 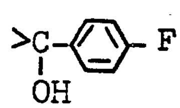 | 4-Cl | 227 | HCl |
| 42 | 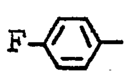 | 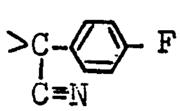 | 4-Cl | 258 | HCl |
| 43 | 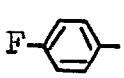 | >CO | 4-Cl+3-$CF_3$ | 183 | $CH_3SO_3H$ |
| 44 | 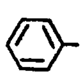 | >CO | 4-Cl+3-$CF_3$ | 202 | $CH_3SO_3H$ |
| 45 | 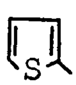 | >CO | 4-Cl+3-$CF_3$ | 240 | HCl |

| Beispiel | Ar | X | R | Schemlzpkt.: °C | Salz |
|---|---|---|---|---|---|
| 46 | 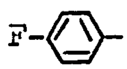 | 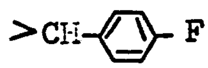 | 4-Cl+3-$CF_3$ | 117 | Base |
| 47 | 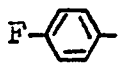 | -O- | 4-Cl+3-$CF_3$ | 189 | $CH_3SO_3H$ |
| 48 | 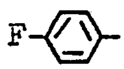 | -S- | 4-Cl+3-$CF_3$ | 170 | $CH_3SO_3H$ |
| 49 | 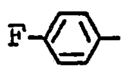 | -NH- | 4-Cl+3-$CF_3$ | 184 - 185 | 2HCl |
| 50 | 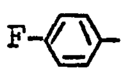 | >CH-C≡N | 4-Cl+3-$CF_3$ | 120 | HCl |
| 51 | 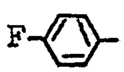 | >CH-OH | 4-Cl+3-$CF_3$ | 238 | HCl |
| 52 | 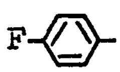 | 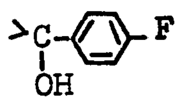 | 4-Cl+3-$CF_3$ | 145 - 147 | HCl |
| 53 | 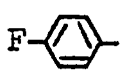 | 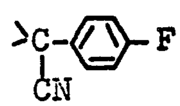 | 4-Cl+3-$CF_3$ | 300 | HCl |
| 54 | 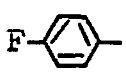 | >CO | 3-$CF_3$ | 219 | $CH_3SO_3H$ |
| 55 | 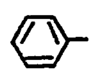 | >CO | 3-$CF_3$ | 247 - 248 | HCl |
| 56 | 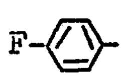 | 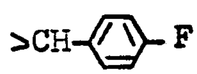 | 3-$CF_3$ | 123 - 125 | Base |
| 57 | 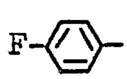 | -O- | 3-$CF_3$ | 194 | $CH_3SO_3H$ |
| 58 | 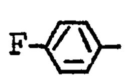 | -S- | 3-$CF_3$ | 192 | $CH_3SO_3H$ |
| 59 | 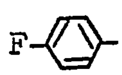 | -NH- | 3-$CF_3$ | 202 - 203 | 2HCl |
| 60 | 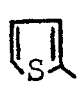 | >CO | 3-$CF_3$ | 176 | $CH_3SO_3H$ |

| Beispiel | Ar | X | R | Schmelzpkt.:°C | Salz |
|---|---|---|---|---|---|
| 61 | 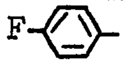 | >CH-C≡N | 3-$CF_3$ | 172 - 173 | $CH_3SO_3H$ |
| 62 | 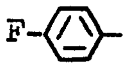 | >CH-OH | 3-$CF_3$ | 231 | HCl |
| 63 | 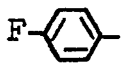 | 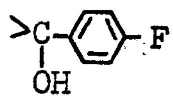 | 3-$CF_3$ | 207 | HCl |
| 64 | 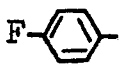 | 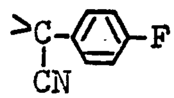 | 3-$CF_3$ | 285 | HCl |
| 65 | 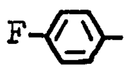 | >CO | 4-$CH_3$ | 203 | $CH_3SO_3H$ |
| 66 | 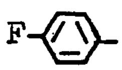 | >CH-C≡N | 4-$CH_3$ | 194 | $CH_3SO_3H$ |
| 67 | 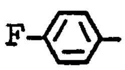 | 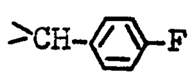 | 4-$CH_3$ | 227 | $CH_3SO_3H$ |
| 68 | 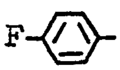 | -O- | 4-$CH_3$ | 215 | $CH_3SO_3H$ |
| 69 | 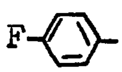 | -S- | 4-$CH_3$ | 181 | $CH_3SO_3H$ |
| 70 | 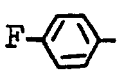 | -NH- | 4-$CH_3$ | 212 | 2HCl |
| 71 | 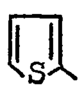 | >CO | 4-$CH_3$ | 205 | $CH_3SO_3H$ |
| 72 | 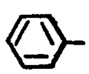 | >CO | 4-$CH_3$ | 180 | $CH_3SO_3H$ |
| 73 | 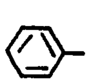 | >CO | 4-$OCH_3$ | 143 | $CH_3SO_3H$ |
| 74 | 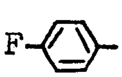 | >CO | 4-$OCH_3$ | 166 | $CH_3SO_3H$ |
| 75 | 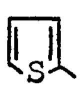 | >CO | 4-$OCH_3$ | 184 | $CH_3SO_3H$ |

| Beispiel | Ar | X | R | Schmelzpkt.: °C | Salz |
|---|---|---|---|---|---|
| 76 | 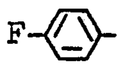 | >CH-C≡N | 4-$OCH_3$ | 152 | $CH_3SO_3H$ |
| 77 | 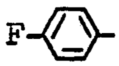 | 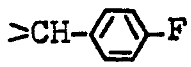 | 4-$OCH_3$ | 195 | $CH_3SO_3H$ |
| 78 | 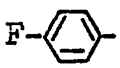 | -O- | 4-$OCH_3$ | 177 | $CH_3SO_3H$ |
| 79 | 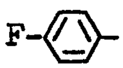 | -S- | 4-$OCH_3$ | 172 | $CH_3SO_3H$ |
| 80 | 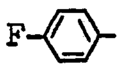 | -NH- | 4-$OCH_3$ | 191 | 2HCl |
| 81 | 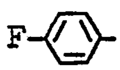 | >CH-OH | 4-$OCH_3$ | 130 | HCl |
| 82 | 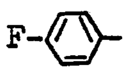 | 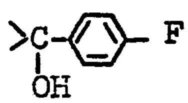 | 4-$OCH_3$ | 207 | HCl |
| 83 | 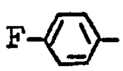 | 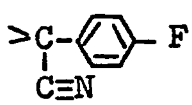 | 4-$OCH_3$ | 219 | HCl |

Beispiel 84 (Verfahren b)

N-(4,4-Bis-p-Fluorphenylbutyl)-3-(4-fluorphenyl)-3-nortropanol-methansulfonat

Unter Stickstoff werden 50 ml einer 0,16 molaren Butyllithium-Hexanlösung in 100 ml Äther vorgelegt und bei -35°C mit 14,9 g (0,085 Mol) p-Fluorbrombenzol in 50 ml Äther innerhalb von 10 min versetzt. Nach weiteren 5 min kräftigem Rühren bei -35°C gibt man 14,8 g (0,04 Mol) N-(4,4-Bis-4'-Fluorphenylbutyl)-nortropinon in 70 ml Äther schnell dazu und läßt 1 Stunde bei dieser Temperatur nachreagieren. Anschließend wird die Lösung auf 100 ml Wasser unter Zugabe von 50 g Ammoniumchlorid gegeben. Die Ätherphase wird dreimal mit je 50 ml Wasser gut gewaschen, getrocknet über Magnesiumsulfat und eingeengt. Der Rückstand wird in 200 ml Petroläther suspendiert und die Lösung abdekantiert. Der Rückstand löst sich in Äthanol auf. Zugabe von molarer Menge Methansulfonsäure in Äthanol kristallisiert das Methansulfonat obiger Verbindung, das aus wenig Äthanol umkristallisiert wird. Man erhält 12,4 g in einer Ausbeute von 55,1 % mit einem Schmelzpunkt von 226°C (identisch mit Beispiel 25).

| | | | | | |
|---|---|---|---|---|---|
| $C_{19}H_{30}F_3NO$ x $CH_3SO_3H$ | ber. | C 64,15 | H 6,10 | F 10,15 | N 2,49 |
| (MW: 561,65) | gef. | 64,37 | 6,12 | 9,84 | 2,36 |

Beispiel 85: (Verfahren c)

N-(4,4-Bis-4'-Fluorphenyl-4-hydroxybutyl)-3-(4-fluorphenyl)-3-nortropanolhydrochlorid

Unter Stickstoff werden 50 ml (0,08 Mol) n-Butyllithium/Hexan-Lösung in 100 ml absolutem Äther vorgelegt. Dazu tropft man bei -35°C 14,9 g (0,085 Mol) p-Fluorbrombenzol in 50 ml Äther innerhalb von 10 Minuten dazu. Nach weiteren 5 Minuten werden 7,2 g (0,025 Mol) N-(4-Fluorphenyl-4-oxobutyl)-nortropinon-Base in 30 ml Äther innerhalb von 5 Minuten schnell dazugetropft. Bei -40 bis -25°C läßt man 1 Stunde nachreagieren. Die Suspension wird auf 100 ml Wasser, dem 40 g Ammoniumchlorid zugefügt sind, gegossen. Die Ätherphase wird abgetrennt, dreimal mit je 50 ml Wasser gewaschen, getrocknet und eingeengt. Der Rückstand wird in 200 ml Petroläther (Kp.: 40 - 60°C) verrührt und abdekantiert. Der Rückstand wird in Äthanol gelöst, mit äthanolischer Salzsäure versetzt und durch Zugabe von Äther kristallisiert. Der Rückstand wird mehrmals aus Alkohol/Äther kristallisiert. Man erhält 1,4 g Hydrochlorid der obigen Verbindung in einer Ausbeute von 10,8 % mit einem Schmelzpunkt von 215°C. Die Verbindung ist mit Beispiel 27 identisch.

| | | | | | |
|---|---|---|---|---|---|
| $C_{29}H_{30}F_3NO_2$ x HCl | ber. | C 67,24 | H 6,03 | F 11,00 | N 2,70 |
| (MW: 518,00) | gef. | 67,26 | 6,13 | 10,95 | 2,54 |

C. Pharmazeutische Formulierungsbeispiele

a) Dragées

1 Dragéekern enthält:

| | |
|---|---|
| Wirkstoff gemäß vorliegender Erfindung | 2,0 mg |
| Milchzucker | 28,5 mg |
| Maisstärke | 17,0 mg |
| Gelatine | 2,0 mg |
| Magnesiumstearat | 0,5 mg |
| | 50,0 mg |

Herstellung:

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragées werden mit Bienenwachs poliert.

Dragée-Endgewicht: 100 mg.

b) Tabletten

| | |
|---|---|
| Wirkstoff gemäß vorliegender Erfindung | 2,0 mg |
| Milchzucker | 55,0 mg |
| Maisstärke | 38,0 mg |
| lösliche Stärke | 4,0 mg |
| Magnesiumstearat | 1,0 mg |
| | 100,0 mg |

Herstellung:
Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die 2 mg Wirkstoff enthalten.

c) Suppositorien

1 Zäpfchen enthält:

| | |
|---|---|
| Wirkstoff gemäß vorliegender Erfindung | 1,0 mg |
| Zäpfchenmasse | 1699,0 mg |

Herstellung:
Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) Ampullen

| | | |
|---|---|---|
| Wirkstoff gemäß vorliegender Erfindung | | 2,0 mg |
| Natriumchlorid | | 18,0 mg |
| destilliertes Wasser | ad | 2,0 ml |

Herstellung:
Wirkstoff und Natriumchlorid werden in Wasser gelöst, die Lösung frei von suspendierten Partikeln filtriert und in 2 ccm-Ampullen unter aseptischen Bedingungen abgefüllt. Zuletzt werden die Ampullen sterilisiert und verschlossen.
Jede Ampulle enthält 2 mg Wirkstoff.

## PATENTANSPRÜCHE:

1. 8-Arylalkyl-3-phenyl-3-nortropanole der allgemeinen Formel

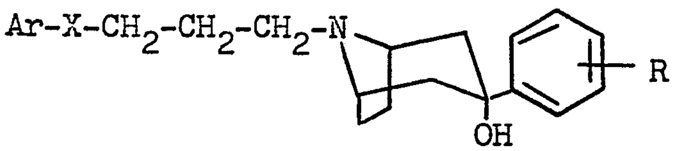

I

worin

Ar einen Rest der Teilformeln 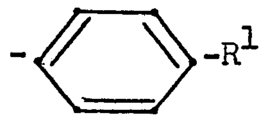 , 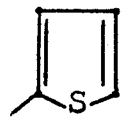 ,

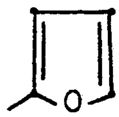 oder 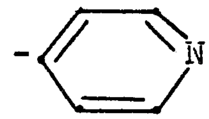

bedeutet,

wobei $R^1$ Wasserstoff, Fluor, Brom, Methyl oder Methoxy ist; und

X die Reste >CO, >CH-CN, >CHOH, 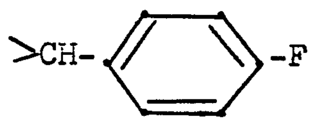 ,

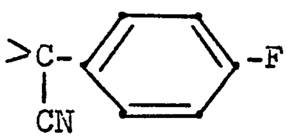 , 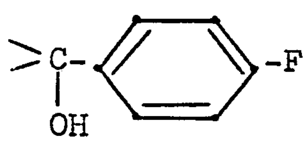 oder -O-, -S- oder

-NH- bedeutet; und

R Wasserstoff oder 4-Fluor, 4-Chlor, 4-Trifluormethyl, 3-Trifluormethyl, 3-Trifluormethyl-4-chlor, 4-Methyl oder 4-Methoxy bedeutet und deren Säureadditionssalze.

2. Verbindungen der allgemeinen Formel I nach Anspruch 1, worin Ar einen 4-Fluorphenylrest,

X die Reste >CO, >CH-CN, >CHOH oder 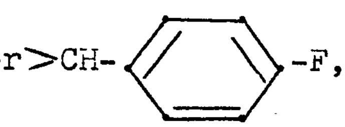 ,

R ein Halogenatom bedeutet, sowie deren Säureadditionssalze.

3. 8-(4-p-Fluorphenyl-4-oxobutyl)-3-(4-chlorphenyl)-3-nortropanol oder dessen Säureadditionssalze.

4. Verfahren zur Herstellung von 8-Arylalkyl-3-phenyl-3-nor-tropanolen der allgemeinen Formel

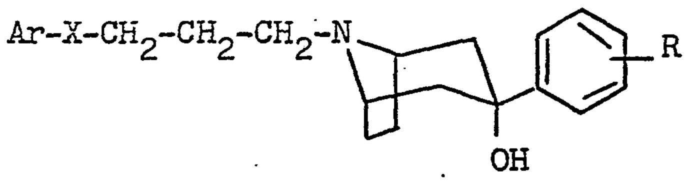

I

worin

Ar einen Rest der Teilformel 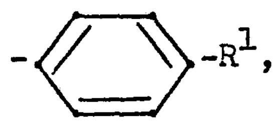, 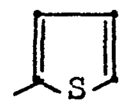,

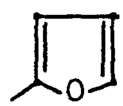 oder 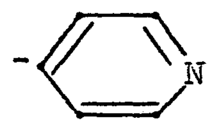

bedeutet,

wobei $R^1$ Wasserstoff, Fluor, Brom, Methyl oder Methoxy ist;

und

X die Reste >CO, >CH-CN, >CH-OH, >CH- 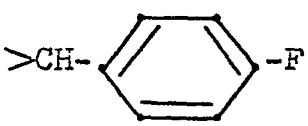 ,

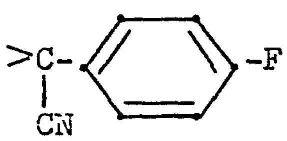 , 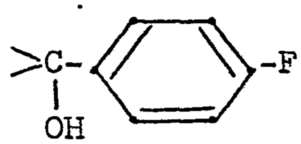 oder -O-, -S- oder -NH-

bedeutet; und

R Wasserstoff oder 4-Fluor, 4-Chlor, 4-Trifluormethyl, 3-Trifluormethyl, 3-Trfluormethyl-4-chlor, 4-Methyl oder 4-Methoxy bedeutet, und von deren Säureadditionssalzen, dadurch gekennzeichnet, daß man

a) ein 3-Phenyl-3-nortropanol der allgemeinen Formel

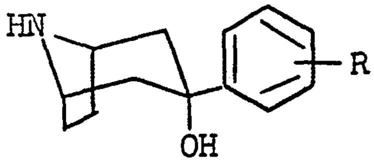

II

worin R die oben genannte Bedeutung hat, mit einem Alkyierungsmittel der allgemeinen Formel

$$Ar\text{-}X\text{-}CH_2\text{-}CH_2\text{-}CH_2\text{-}Y$$ III

umsetzt,
worin Y ein Halogenatom oder einen Alkylbenzolsulfonyloxyrest bedeutet und Ar und X wie oben angegeben definiert sind; oder

b) zur Herstellung von Verbindungen der allgemeinen Formel I, worin X die Gruppen 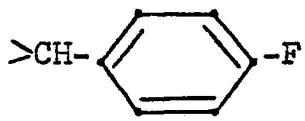 , -O- oder -S- bedeutet,

ein 8-Arylalkyl-nortropin-3-on der allgemeinen Formel

$Ar\text{-}X'\text{-}CH_2\text{-}CH_2\text{-}CH_2\text{-}$ 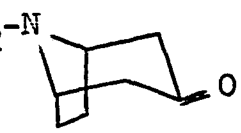 IV

worin X' die Gruppen 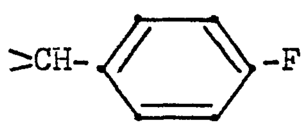, -O- oder -S- bedeutet und

Ar wie oben angegeben definiert ist, mit einer Lithiumphenyl-Verbindung der allgemeinen Formel

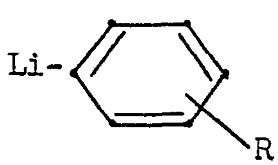

V

umsetzt,
worin R die oben genannte Bedeutung hat; oder

c) zur Herstellung von Verbindungen der allgemeinen Formel I, worin X die Gruppe 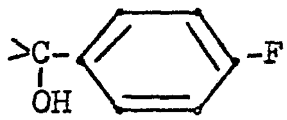 und R 4-Fluor bedeuten, ein 8-Aroylpropyl-nortropin-3-on der allgemeinen Formel

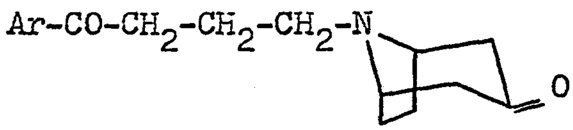 VI

worin Ar die oben angegebene Bedeutung hat, mit mindestens 2 Molen 4-Fluorphenyl-lithium umsetzt; oder

d) zur Herstellung von Verbindungen der allgemeinen Formel I, worin X die CO-Gruppe bedeutet, ein 8-Arylalkyl-3-phenyl-3-nortropanol der allgemeinen Formel

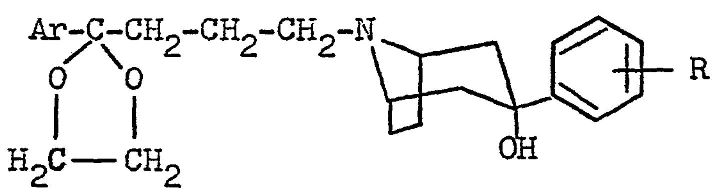 VII

worin Ar und R die oben genannten Bedeutungen besitzen, mit verdünnten Säuren umsetzt; und gegebenenfalls die erhaltenen Verbindungen in ihre Säureadditionssalze überführt.

5. Verfahren nach Anspruch 4, dadurch gekennzeichnet, daß man die Umsetzungen a) und d) in Gegenwart eines säurebindenden Stoffes durchführt.

6. Verfahren nach Anspruch 4 und/oder 5, dadurch gekennzeichnet, daß man die Umsetzung a) bei Temperaturen von 50 bis

150°C, die Umsetzung b) und c) bei Temperaturen von -40 bis -15°C durchführt.

7. Verfahren nach einem der vorhergehenden Ansprüche 4 bis 6, dadurch gekennzeichnet, daß man die Umsetzungen in einem inerten Lösungsmittel vornimmt.

8. Pharmazeutische Zubereitungen, dadurch gekennzeichnet, daß sie eine oder mehrere Verbindungen der Formel I nach Anspruch 1 neben üblichen Hilfs- und Trägerstoffen enthalten.

9. Verfahren zur Herstellung von pharmazeutischen Zubereitungen nach Anspruch 8, dadurch gekennzeichnet, daß man Verbindungen der Formel I nach Anspruch 1 mit üblichen galenischen Hilfs- und Trägerstoffen zu üblichen pharmazeutischen Darreichungsformen verarbeitet.

10. Verbindungen der allgemeinen Formel I nach Anspruch 1 zur Verwendung für die Herstellung von Arzneimitteln mit neuroleptischer Wirkung.

11. Verwendung von Verbindungen der allgemeinen Formel I nach Anspruch 1 zur Herstellung von Arzneimitteln mit neuroleptischer Wirkung.

0053744

Europäisches Patentamt

# EUROPÄISCHER RECHERCHENBERICHT

Nummer der Anmeldung

EP 81109767.4

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| Y | DE - A - 1 174 792 (C.F. BOEHRINGER)<br>* Spalte 1; Spalte 2, Zeile 20 * | 1,4 | C 07 D 451/06<br>A 61 K 31/46 |
| Y | US - A - 2 921 938 (WETTERAU)<br>* Spalten 1,2; Beispiel 21 * | 1,4,8,9 | |
| Y | US - A - 3 657 252 (KAISER et al.)<br>* Spalten 1-4 * | 1,4,8,9 | |

RECHERCHIERTE SACHGEBIETE (Int. Cl.³)

C C7 D 451/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veroffentlicht worden ist
D: in der Anmeldung angefuhrtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 23-02-1982 | PETROUSEK |

EPA form 1503.1 06.78